# EUROPÄISCHE PATENTSCHRIFT

(11) **EP 0 977 736 B1**
(45) Veröffentlichungstag und Bekanntmachung des Hinweises auf die Patenterteilung: **24.09.2003**
(21) Anmeldenummer: 98920511.7
(22) Anmeldetag: 11.04.1998
(51) Int. Cl.: C07D 213/75, A61K 31/44

(54) **VERFAHREN ZUR HERSTELLUNG VON REINEM FLUPIRTIN-MALEAT UND DESSEN MODIFIKATION A**
PROCESS FOR PREPARING PURE FLUPIRTIN MALEATE AND ITS MODIFICATION A
PROCEDE DE PREPARATION DE MALEATE DE FLUPIRTINE PUR ET DE SA MODIFICATION A

(30) Priorität: 23.04.1997 DE 19716984
(43) Veröffentlichungstag der Anmeldung: 09.02.2000
(73) Patentinhaber: AWD.pharma GmbH & Co.KG, 01097 Dresden (DE)
(72) Erfinder: OLBRICH, Alfred, D-33790 Halle (DE); EMIG, Peter, D-63486 Bruchköbel (DE); KUTSCHER, Bernhard, D-63477 Maintal (DE); LANDGRAF, Karl-Friedrich, D-01217 Dresden (DE); PAULUHN, Siegfried, D-74731 Walldürn (DE); STANGE, Hans, D-01587 Riesa (DE)
(86) Internationale Anmeldenummer: EP9802118
(87) Internationale Veröffentlichungsnummer: WO98047872

(56) Entgegenhaltungen:
- DE-A- 3 133 519
- S SCHWOCH ET AL.: "2,3-Dihydrospiro[1H-4- and 5-azabenzimidazole-2,1'-cyclohexane]: Reactions with nucleophiles" HELVETICA CHIMICA ACTA., Bd. 77, Nr. 8, 1994, Seiten 2175-2190, XP002073789 BASEL CH

## Beschreibung

Die vorliegende Erfindung betrifft ein Verfahren zur Herstellung von reinem Flupirtin-Maleat sowie der reinen Kristallmodifikation A des Flupirtin-Maleates.

Flupirtin-Maleat ist 2-Amino-3-carbethoxyamino-6-(p-fluor-benzylamino)-pyridin-mafeat (I). Diese Verbindung befindet sich unter dem Markennamen Katadolon® im Handel und wird insbesondere als Analgetikum eingesetzt.

Die Herstellung von Flupirtin-Maleat wird in DE 31 33 519 beschrieben.

2-Amino-3-nitro-6-(4-fluorbenzyl-amino)-pyridin (ANFP) wird in 2-Methoxyethanol gelöst und bei 5 bar und 60 °C mit Wasserstoff in Gegenwart von Raney-Nickel zu 2,3-Diamino-6-(4-fluorbenzylamino)pyridin hydriert. Dieses wird mit Chlorameisensäureethylester und Triethylamin unter Inertgas zur Flupirtin-Base acyliert. Der Katalysator wird filtriert und in das Filtrat, das Triethylamin Hydrochlorid enthält, direkt eine Lösung von Isopropanol und Maleinsäure gegeben, wobei unter starkem Rühren und Inertgas-Atmosphäre das Flupirtin-Rohmaleat ausfällt.

Seit bekannt wurde, daß das für die Hydrierung von ANFP eingesetzte Lösungsmittel 2-Methoxyethanol aufgrund seines beträchtlichen Gefährdungspotentials im Urogenitalbereich mit einem Gesundheitsrisiko verbunden ist, stellt diese Verbindung ein bedenkliches Solvens für diese Reaktion im technischen Maßstab dar.

Bei der Ausfällung des Flupirtin-Maleates aus der zugrundeliegenden Base ist die Vermeidung störender, durch Luftsauerstoff induzierter Farbkomplexe von entscheidender Bedeutung. Aufgrund ihrer Farbintensität können sie die weitere Reinigung bis hin zum spezifikationsgerechten Flupirtin-Maleat sehr stark beeinträchtigen.

Es ist bei diesem Reaktionsschritt daher der Ausschluß von Luftsauerstoff durch eine intensive Inertgaszuführung und ein geschlossenes Reaktorsystem dringend erforderlich.

In einem sehr zeitintensiven und umständlichen Reinigungsverfahren wird Flupirtin-Rohmaleat durch Freisetzung mit Ammoniak bzw. Natronlauge in Flupirtin-Rohbase überführt. Bei diesem Freisetzungsprozeß entstehen abwasserbelastende Ammonium- beziehungsweise Natrium-Salze, die eine störende Begleiterscheinung aus ökologischer Sicht bedeuten. Die Fiupirtin-Rohbase wird aus Isopropanol umkristallisiert und nach einer Aktivkohle / Kieselgur-Klärung mit einer isopropanolischen Maleinsäure-Lösung zu Flupirtin-Reinmaleat umgesetzt.

Das bisherige aufwendige Verfahren läßt sich durch folgendes Reaktionsschema verdeutlichen:
- A:: ANFP → Hydrierung → Acylierung → Flupirtin-Rohbase I
- B:: Flupirtin-Rohbase 1 → Maleinsäure → Flupirtin-Rohmaleat
- C:: Flupirtin-Rohmaleat → Freisetzung → Flupirtin-Rohbase 11
- D:: Flupirtin-Rohbase 11 → Umkristallisation → Flupirtin-Reinbase
- E:: Flupirtin-Reinbase → Maleinsäure → Modifizierung → Flupirtin-Reinmaleat
wobei die Reaktionsschritte C - E erforderliche Reiniungsschritte sind, ohne die kein reines, einheitliches und spezifikationsgerechtes Reinmaleat erhalten werden konnte. Sie stellen sehr arbeits- und kostenintensive Reinigungsoperationen dar, verlängern den Syntheseprozeß erheblich und führen nur auf einem produktionstechnisch sehr aufwendigen, komplizierten und umständlichen Weg zu einem spezifikationsgerechten Flupirtin-Reinmaleat.

Als ein weiteres Kriterium für einen technisch sehr hohen Aufwand bei den Reinigungsschritten C und E gelten die extrem großen Reaktorvolumina, die zur Kristallisation der Flupirtin-Base und zur Fällung des Flupirtin-Maleates erforderlich sind und ein erhebliches Maß an Produktionskapazität binden.

Pro Tonne Flupirtin-Maleat müssen 25 t Abfall-Lösung die 2-Methoxyethanol, Isopropanol, Ammoniak, Ammoniummaleat und Wasser enthalten, durch Verbrennung entsorgt werden.

Diese Verfahrensweise ist in höchstem Maße unwirtschaftlich, schwierig zu handhaben und entspricht auch nicht den Anforderungen an eine umweltverträgliche Herstellung.

Das Flupirtin-Maleat wird im allgemeinen als ein Gemisch von 2 Kristallmodifikationen A und B, wobei diese in unterschiedlichen Verhältnissen im Gemisch vorliegen, erhalten.

Für die galenische Weiterverarbeitung stellen derartige Mischungen ein großes Problem dar. Insbesondere wirkt sich das nachteilig auf die Einhaltung konstanter galenischer Herstellungsbedingungen und damit auf die Gewährleistung der pharmazeutischen Qualität eines Wirkstoffes aus. Diese unterschiedlichen Kristallmodifikations-Gemische führen im Verlaufe der Freisetzung aus der galenischen Zubereitung im menschlichen Organismus zu unterschiedlichen Freisetzungsgeschwindigkeiten. Dieser Mangel kann den Wirkungseintritt des Flupirtinmaleates als Wirkstoff verzögern und somit die Wirkungsbilanz verfälschen.

Es stellt sich also die Aufgabe, ein einfaches, umweltverträgliches Verfahren zur Herstellung von reinem Flupirtin-Maleat zu entwickeln aus dem durch Kristallmodifizierung die reine Modifikation A erhalten werden kann.

Erfindungsgemäß wird die Aufgabe dadurch gelöst, daß die Hydrierung von 2-Amino-3-nitro-6-(4-fluorbenzyl-amino)-pyridin (ANFP) in Gegenwart von Raney-Nickel, Acylierung der Zwischenverbindung 2,3-Diamino-6-(4-fluorbenzylamino)- pyridin (DAFP) mit Chlorameisensäureethylester und die Umsetzung der erhaltenen Flupirtin-Base mit Maleinsäure in wasserlöslichen Alkoholen, wie Ethanol oder Isopropanol erfolgt.

Dabei sind folgende Verfahrensvarianten möglich:
1. Verfahrensvariante:
   - A:: ANFP → Hydrierung → Acylierung → Flupirtin-Rohbase
   - B:: Flupirtin-Rohbase → Maleinsäure → Flupirtin-Rohmaleat
   - C - E:: entfällt
   - F:: Rohmaleat → Reinmaleat

   Die im Verfahrensschritt A in situ synthetisierte, gelöste und sehr sauerstoffempfindliche Rohbase konnte durch einen sehr raschen Absaugprozeß in eine wäßrige Maleinsäure-Lösung überführt werden, aus der unter Rühren sofort das gegenüber früher weitaus weniger gefärbte Flupirtin-Rohmaleat ausfällt. Dieses lieferte nach Kristallisation in Isopropanol - Wasser ein spezifikationsgerechtes Reinmaleat in etwa 85 %-iger Ausbeute.
   Mit dieser gegenüber dem bisher praktizierten Verfahren unter Umgehung der Verfahrensschritte C-E drastischen Abkürzung des Herstellprozesses für Flupirtin-Maleat gelingt es, aus dem Flupirtin-Rohmaleat direkt das Reinmaleat zu gewinnen und die störende Farbproblematik der Flupirtinherstellung schon auf einer frühen Stufe auszuschalten.
2. Verfahrensvariante:
   - A:: ANFP → Hydrierung → Acylierung → Fiupirtin-Rohbase
   - B:: Flupirtin-Base → Maleinsäure → Flupirtin-Rohmaleat
   - C - F:: entfallen
   - G:: ohne Isolierung des Rohmaleates → Reinmaleat

   Der Verfahrensschritt G stellt gegenüber Verfahrensschritt F eine noch deutlich verkürztere Verfahrensalternative dar, bei der die Flupirtin-Rohmaleat-Fällung aus der in isopropanol in situ gebildeten Flupirtin-Base durch Absaugen in eine wäßrige Maleinsäure-Lösung bei 50-60 °C erfolgt und nach Kristallmodifizierung farbloses Flupirtin-Reinmaleat in 85-90 % -iger Ausbeute erhalten wird.
3. Verfahrensvariante:
   - A:: ANFP → Hydrierung → Acylierung → Flupirtin-Rohbase (isoliert)
   - B:: Flupirtin-Reinbase → Maleinsäure → Flupirtin-Reinmaleat

   Hierbei wird die Flupirtin-Base nach erfolgter Acylierung bevorzugt in Ethanol oder Wasser gefällt und durch einfache Umkristallisation gereinigt. Die Herstellung des Flupirtin-Reinmaleates erfolgt dann analog zu den genannten Verfahrensvarianten.

Es war völlig überraschend, daß sowohl die Hydrierung von ANFP als auch die Acylierung des gebildeten DAFP's mit Chlorameisensäureethylester in wasserlöslichen Alkoholen, wie Ethanol oder Isopropanol durchgeführt werden kann.

Hierbei erhält man bei Fällung in wäßriger Maleinsäurelösung ein nickelfreies Flupirtin-Maleat, welches keinem aufwendigen, mehrstufigen Reinigungsprozeß mehr unterzogen werden muß.

Als weiterer Vorteil stellte sich heraus, daß erstmals bei der Flupirtin-Rohmaleat-Fällung ein von Farbkomplexen freies, weißes Produkt erhalten wurde, so daß auch hier keine zusätzlichen Reinigungsoperationen mehr erforderlich sind.

Für die anschließende Kristallmodifzierung zum Erhalt der reinen Modifikation A des Flupirtin-Maleats wurde völlig überraschend gefunden, daß Mischungen der beiden Modifikationen A und B des Flupirtin-Maleates unter bestimmten Bedingungen durch Rühren in phasenreines, nur aus der Modifikation A bestehendes Flupirtin-Maleat umgewandelt werden können.

Als besonders vorteilhaft erwiesen sich dabei ein hoher Anteil der Modifikation A in der Ausgangsprobe, eine hohe Feststoffkonzentration in der Suspension (1 : 1 bis 1 : 0,8) und Temperaturen im Bereich von - 10 bis 60 °C.

Bei hoher Feststoffkonzentration (1:1) und Temperaturen zwischen 20 und 60 °C konnte jedoch auch aus überwiegend der Modifikation B bestehendes Flupirtin-Maleat (90% B) bei Rührzeiten von 2 bis 5 Stunden vollständig in die Modifikation A umgewandelt werden, wobei als besonders überraschendes Vorteilsargument gefunden wurde, daß die Einstellung der gewünschten Kristallmodifikation A auch bei Temperaturen von 0 bis 30 °C erzielt werden kann.

Überträgt man diese Erkenntnisse auf den Fällungsprozeß (Zusammenführen von Flupirtin-Base und Maleinsäure in einem geeigneten Lösungsmittel), dann wird man zunächst die nach dem Stand der Technik zur Erzielung eines möglichst hohen primären Anteils der Modifikation A erforderlichen Bedingungen einhalten, dann jedoch durch einen entsprechend geführten Rührprozeß die vollständige Umwandlung vorhandener B-Anteile in die Modifikation A gewährleisten.

Zweckmäßigerweise wird sich der Rührprozeß unmittelbar an die Fällung anschließen, das heißt, man wird, beginnend während der Abkühlung, das Rühren auch nach der Abkühlung auf Raumtemperatur so lange fortsetzen bis die Umwandlung vollständig ist. Dabei wird man die Feststoffkonzentration so hoch wie möglich halten.

Überraschenderweise wurden bei der Umkristallisation und Fällung sowohl in Isopropanol als auch in Ethanol bisher nicht bekannte kristallisierte Solvate des Flupirtin-Maleates gefunden.

Die in den Kristallsuspensionen beobachteten Solvate werden während des Rührprozesses in die Modifikation A umgewandelt. Bei der Dispergierung vorher bis zur Gewichtskonstanz getrockneter Kristallisate in Isopropanol, Ethanol oder Wasser wird dagegen keine Solvat- oder Hydratbildung beobachtet. Die Solvate wurden nur bei der primären Kristallisation (Keimbildung) beobachtet.

Weiterhin wurde gefunden, daß vorgetrocknetes, von äußerlich adsorbiertem Lösungsmittel befreites Kristallisat, das aber noch innerkristallin gebundenes Lösungsmittel enthält, durch Erhitzen auf Temperaturen von etwa 80 - 100 °C in die Modifikation B umgewandelt werden kann. Die nachträgliche Bildung der Modifikation B aus Solvat während der technischen Trocknung wird durch hinreichend langes Rühren der Kristallsuspension nach der Maleatfällung verhindert.

### Phasenanalyse

Die kristallisierten Phasen des Flupirtin-Maleates, Modifikation A und B sowie isopropanol- und Ethanolsolvat wurden durch Röntgenbeugung mit einem Pulverdiffraktometer bestimmt. Bei dieser Methode erhält man ein transformiertes Abbild des oder der vorliegenden Kristallstrukturen, das durch Summation der Beugungseffekte an einer sehr großen Zahl von Kristallen entsteht. Deshalb ist dieses Verfahren für die Bestimmung der Zusammensetzung von aus unterschiedlichen kristallisierten Phasen bestehenden Gemengen besonders geeignet. Mit diesem Verfahren können auch frische, noch lösungsmittelfeuchte Proben untersucht werden. Weiterhin können mit Phasenübergängen verbundene Strukturänderungen direkt beobachtet werden.

### Methode: Röntgendiffraktometrie

### Diffraktogramme der Modifikationen A und B

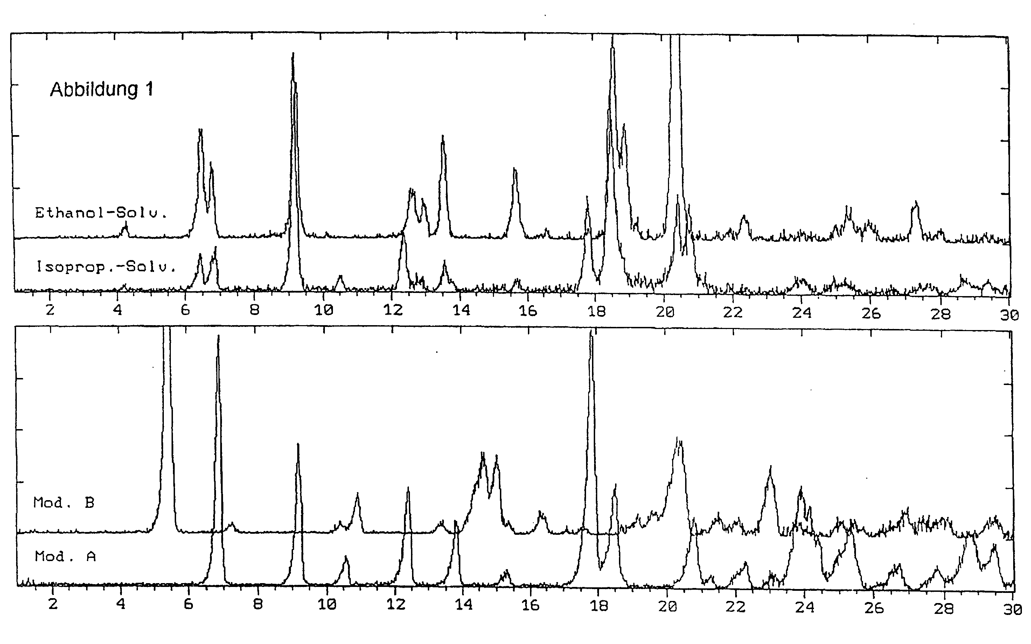

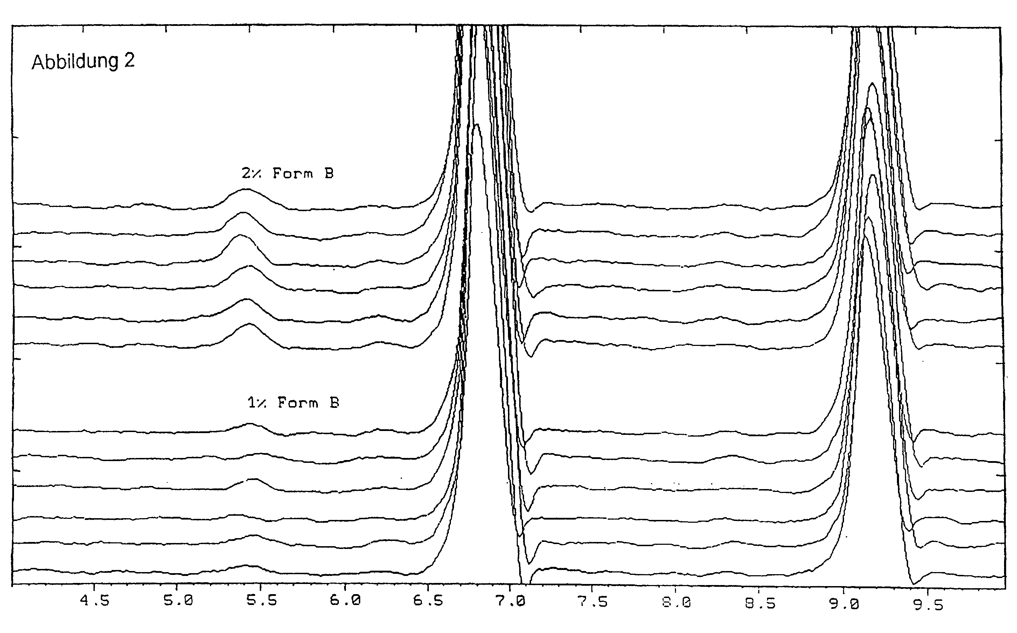

Die Abbildung 1 zeigt im unteren Teil die Diffraktogramme der Modifikationen A und B, Tabelle 1 die Pulverdaten. Beide Modifikationen zeigen charakteristische Reflexe, die nicht mit einem Reflex der jeweils anderen Modifikation koinzidieren. Es sind dies bei der Modifikation A insbesondere die starken Reflexe bei 6.9, 9.2 und 17.9 °2ϑ und bei der Modifikation B insbesondere der stärkste Reflex bei 5.5 °2ϑ. Die Nachweisgrenze für die Modifikation B liegt bei ≤ 1% (Abbildung 2).

**Tabelle 1:**

| Pulverdaten der Modifikationen von Flupirtinmaleat | | | | | |
|---|---|---|---|---|---|
| **Modifikation A** | | | **Modifikation B** | | |
| **20** **(°)** | **d-Wert** **(Å)** | **Intensität** **(%)** | **20** **(°)** | **d-Wert** **(Å)** | **Intensität** **(%)** |
| | | | 5.46 | 16.2 | 100 |
| 6.90 | 12.8 | 98 | | | |
| | | | 7.28 | 12.1 | 3 |
| 9.22 | 9.6 | 55 | | | |
| 10.57 | 8.36 | 11 | 10.43 | 8.48 | 3 |
| | | | 10.91 | 8.10 | 10 |
| 12.39 | 7.14 | 38 | | | |
| | | | 13.42 | 6.59 | 3 |
| 13.81 | 6.41 | 25 | | | |
| | | | 14.44 | 6.13 | 13 |
| | | | 14.66 | 6.04 | 20 |
| | | | 15.04 | 5.89 | 20 |
| 15.32 | 5.78 | 7 | 15.40 | 5.75 | 2 |
| | | | 16.37 | 5.41 | 4 |
| | | | 17:60 | 5.04 | 1 |
| 17.85 | 4.97 | 100 | | | |
| 18.50 | 4.79 | 39 | | | |
| | | | 18.70 | 4.74 | 3 |
| | | | 19.16 | 4.63 | 5 |
| | | | 19.58 | 4.53 | 6 |
| | | | 20.08 | 4.42 | 13 |
| | | | 20.34 | 4.36 | 24 |
| | | | 20.44 | 4.34 | 24 |
| 20.81 | 4.27 | 28 | | | |
| 21.26 | 4.18 | 5 | | | |
| | | | 21.52 | 4.13 | 6 |
| 21.92 | 4.05 | 5 | | | |
| 22.30 | 3.99 | 11 | 22.11 | 4.02 | 5 |
| 23.03 | 3.86 | 7 | 23.02 | 3.86 | 17 |
| 23.90 | 3.72 | 40 | 23.84 | 3.73 | 2 |
| 24.15 | 3.68 | 32 | | | |
| 24.41 | 3.64 | 20 | | | |
| 24.98 | 3.56 | 15 | 25.07 | 3.55 | 3 |
| 25.35 | 3.51 | 27 | 25.48 | 3.49 | 4 |
| 26.68 | 3.34 | 9 | | | |
| | | | 26.97 | 3.30 | 7 |
| | | | 27.48 | 3.24 | 4 |
| 27.81 | 3.21 | 10 | 28.00 | 3.19 | 5 |
| 28.71 | 3.11 | 23 | | | |
| 29.51 | 3.03 | 18 | 29.45 | 3.03 | 3 |
| | | | 30.28 | 2.95 | 3 |
| 30.55 | 2.92 | 17 | | | |

### Diffraktogramme der Solvate

Abbildung 1 zeigt im oberen Teil die beiden Solvate des Flupirtin-Maleates.

Die Diffraktogramme der Solvate unterscheiden sich von den Modifikationen A und B insbesondere durch das Auftreten zusätzlicher Reflexe. Solvat ist neben den beiden Modifikationen insbesondere an dem Reflex bei 6.4 °2ϑ (d=13.7 Å) zu erkennen. Die Modifikation B ist neben Solvat nachweisbar, während kleine Anteile der Modifikation A neben Solvat nicht erkannt werden.
Die Verwandtschaft zwischen den Diffraktogrammen der Solvate und dem Diffraktogramm der Modifikation A spricht dafür, daß der innerkristalline Lösungsmitteleinbau während der Keimbildung der Modifikation A erfolgt.

### Thermische Reaktionen der Kristallphasen

Methoden:
- Differentialscanningcalorimetrie (DSC)
- Thermogravimetrie (TG)
- Temperaturkontrollierte Röntgendiffraktometrie

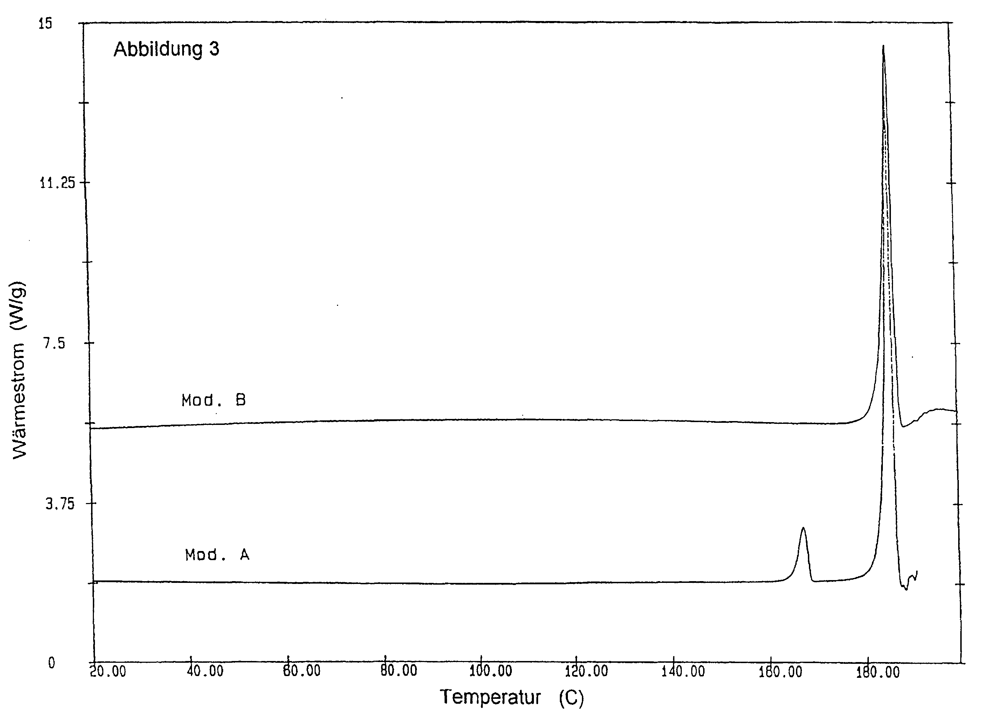

Die Abbildung 3 zeigt die DSC-Kurven der beiden Modifikationen. Die Modifikation A zeigt zwei endotherme Reaktionen und zwar den Umwandlungspunkt A → B bei 164 °C (onset) und den Schmelzpunkt der Modifikation B bei 184 °C (onset). Modifikation B zeigt nur einen endothermen Effekt bei 184 °C, der das Schmelzen anzeigt.

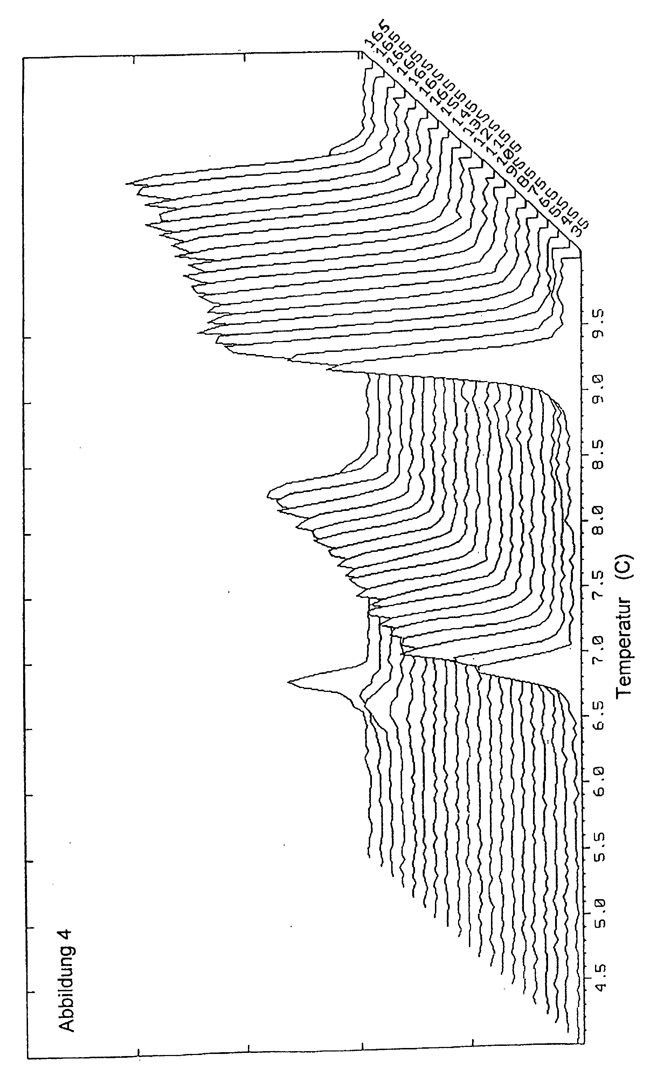

Die Abbildung 4 zeigt die thermische Gitterumwandlung A → B. In Übereinstimmung mit dem Ergebnis der DSC erscheint bei 165 °C der starke Reflex der Modifikation B bei 5.5 °2ϑ.

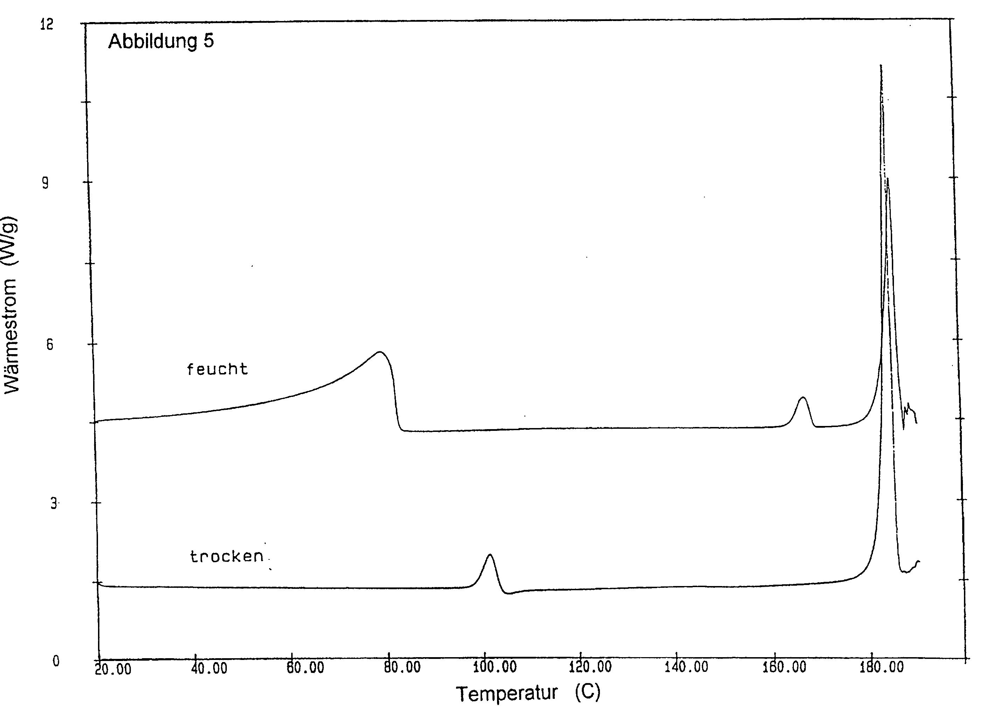

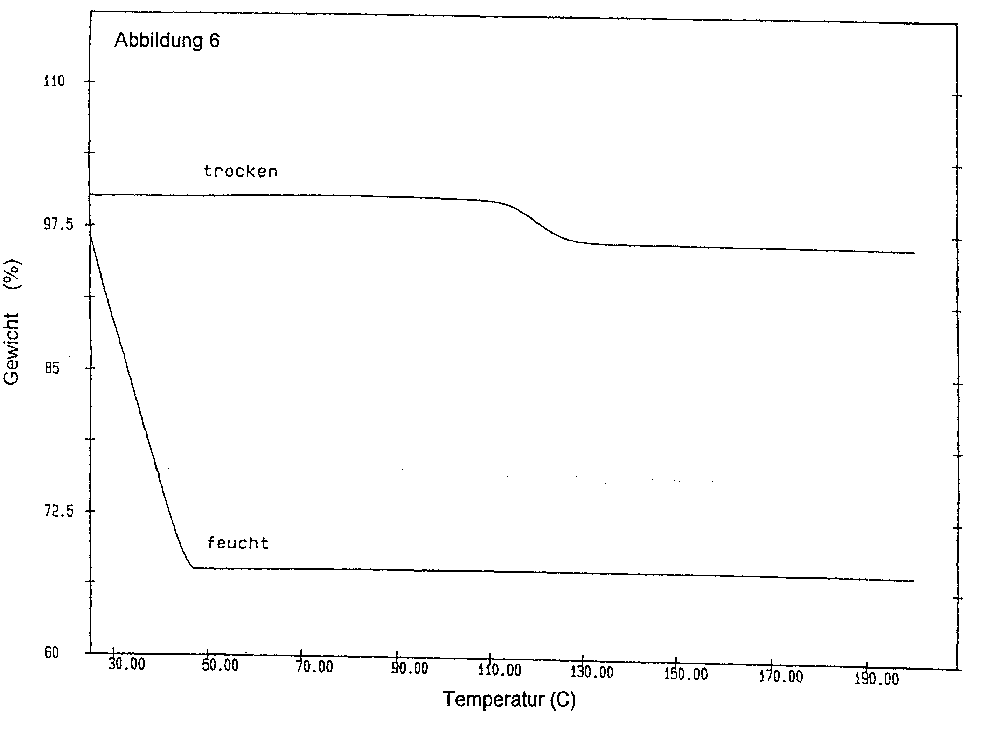

Die Abbildungen 5 und 6 zeigen den unterschiedlichen Verlauf der Kristallreaktionen bei der Erhitzung von lösungsmittelfeuchtem und vorgetrocknetem Kristallisat am Beispiel eines Isopropanolkristallisates. Im feuchten Zustand erfolgt die Desolvatatiön zusammen mit der Abgabe des äußerlich gebundenen Lösungsmittels unter Bildung der Modifikation A, die dann bei 164 °C in die Modifikation B umgewandelt wird

Im trockenen Zustand erfolgt die Dehydratation bei deutlich höherer Temperatur unter Bildung der Modifikation B.

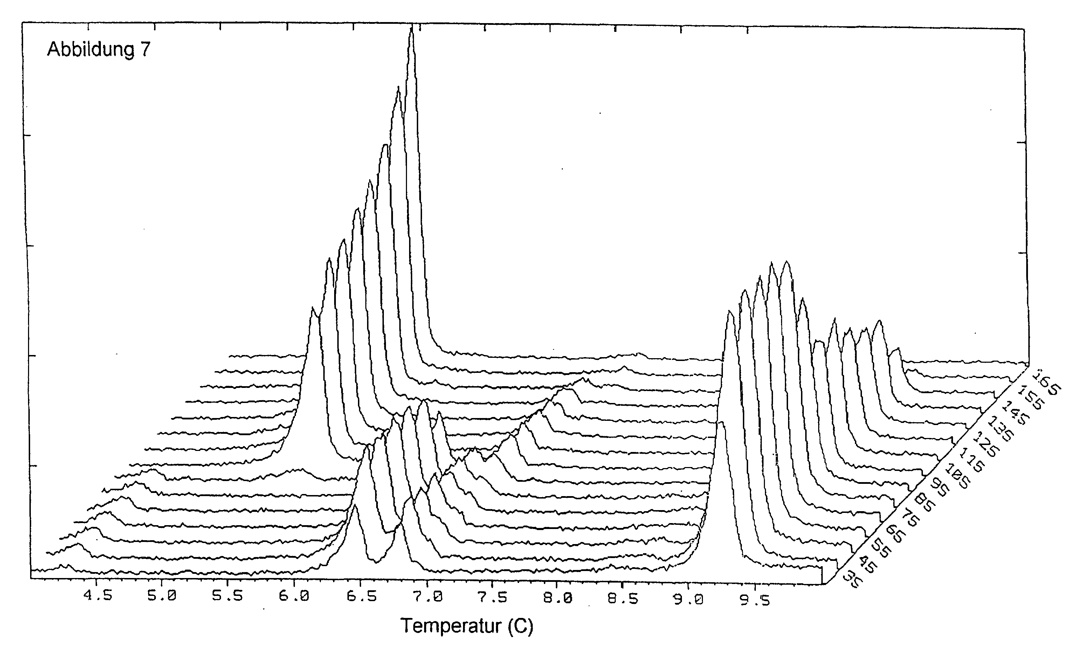

Die Abbildung 7 zeigt die thermischen Gitterumwandlungen bei einem Isopropanolkristallisat. Zunächst liegt Solvat (6.4 °2ϑ) neben der Modifikation A vor. Bei 95 °C verschwindet der charakteristische Solvatreflex und es erscheint der charakteristische Reflex der Modifikation B bei 5.5 °2ϑ. Beim weiteren Erhitzen verschwinden bei starker Intensitätszunahme des B-Reflexes auch die charakteristischen Reflexe der Modifikation A bei 6.9 und 9.2 °2ϑ.

Das erfindungsgemäße Verfahren zur Herstellung von reinem Flupirtin-Maleat und der reinen Modifikation A des Flupirtin-Maleates hat folgende entscheidende Vorteile:
- Im Verfahrensschritt A wird das bedenkliche Lösungsmittel 2-Methoxyethanol gegen wasserlösliche Alkohole, wie Isopropanol oder Ethanol ersetzt.
- Im gleichen Verfahrensschritt kann die Reaktionszeit der N-Acylierung von 2 auf ½ Stunde verringert werden mit dem Ergebnis einer Reduzierung der Nebenproduktpalette und der Konzentration an störenden Farbkomplexen.
- Es entfallen die aufwendigen Reinigungsschritte C - E des bisherigen Flupirtin-Verfahrens. Das Rohmaleat kann durch einen problemlosen Reinigungsvorgang in das Flupirtin-Reinmaleat überführt werden beziehungsweise die Herstellung des Flupirtin-Reinmaleates erfolgt direkt aus der in situ gebildeten Flupirtin-Base.
- Die Temperatur bei der Einstellung der reinen Kristallmodifikation konnte von einem Bereich von 60 bis 65 °C auf ein Intervall von -10 bis 60 °C gesenkt werden.
- Es konnte erstmals die reine Modifikation A des Flupirtin-Maleats hergestellt werden.

Anhand von Beispielen sollen die erfindungsgemäßen Verfahren näher erläutert werden:

### 1. Beispiel

### Herstellung des Flupirtin-Reinmaleates

75 g (0,286 Mol) ANFP werden in einer Suspension von 12,5 g Raney-Nickel in 400 ml Isopropanol bei 65 °C und 5 bar Wasserstoffdruck hydriert. Nach beendeter Hydrierung wird die Lösung mit 26,4 ml Chlorameisensäureethylester und danach mit 50,6 ml Triethylamin versetzt. Nach Zugabe von weiteren 6,3 ml Chlorameisensäureethylester wird die Reaktionslösung 1 Stunde bei 60 °C nachgerührt. Sodann saugt man die heiße Lösung unter Rühren in eine auf 50 - 60 °C erwärmte Lösung von 53,3 g Maleinsäure in 1,5 l H₂O und wäscht den Katalysator mit wenig Isopropanol nach.

Das Flupirtin-Maleat fällt farblos aus, die Kristallsuspension wird unter weiterem Rühren auf 20 °C gekühlt und 20 Minuten bei dieser Temperatur belassen. Man saugt ab, wäscht mit ca. 500 ml Wasser nach und trocknet das Flupirtin-Maleat im Vakuum bei 35 °C.

Ausbeute: 107,55 g (89,6 % d. Theorie, bezogen auf eingesetztes ANFP)

### 2. Beispiel

### Herstellung des Flupirtin-Reinmaleates

18,5 g (0,07 Mol) ANFP werden analog zum Beispiel 1 in einer Suspension von 2,0 g Raney-Nickel in 140 ml Ethanol bei 60 - 70 °C und 5 bar Wasserstoffdruck hydriert. Nach beendeter Hydrierung erfolgt die weitere Umsetzung bei 40 - 50 °C mit 9,3 g Chlorameisensäureethyiester (0,86 Mol) und 9,2 g Triethylamin (0,91 Mol). Die vom Katalysator abgetrennte Reaktionslösung wird unter Rühren in 540 ml Wasser eingetragen. Nach 2 Stunden Rühren bei Raumtemperatur saugt man die ausgefallene Base ab, wäscht mit Wasser und Isopropanol und kristallisiert in der 3,7-fachen Menge Isopropanol um. Ausbeute: 18,4 g (86,0 % d. Theorie)

Die Fällung und Modifizierung von Flupirtin-Reinmaleat erfolgt gemäß der Beispiele 7 und 8.

### 3. Beispiel

### Herstellung von der reinen Modifikation A des Flupirtin-Maleates

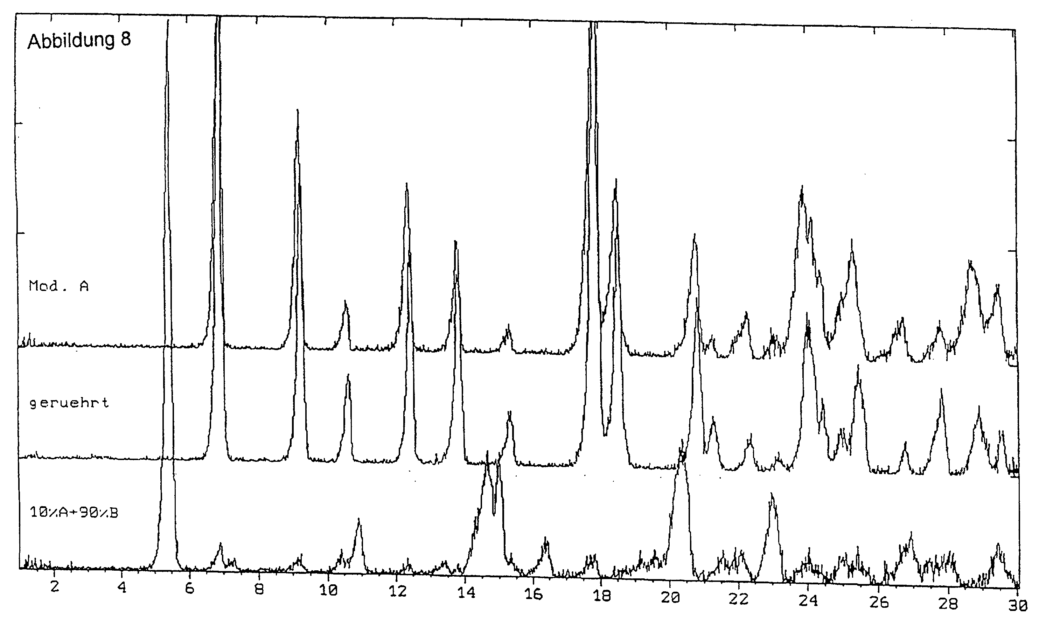

Getrocknetes, aus Isopropanol kristallisiertes Flupirtin-Maleat, das 10 % Modifikation A neben 90 % Modifikation B enthielt (Abbildung 8, unterste Kurve), wurde im Verhältnis 1 : 0,8 in Isopropanol dispergiert.
Nach 200 Minuten Rühren bei 20 °C war der charakteristische starke B-Reflex bei 5.5 °2ϑ verschwunden, und es wurden nur noch die charakteristischen Reflexe der Modifikation A bei 6.9 und 9.2 °2ϑ beobachtet (Abbildung 8. mittlere Kurve)

### 4. Beispiel

Getrocknetes, aus Isopropanol kristallisiertes Flupirtin-Maleat, das 10 % Modifikation A neben 90 % Modifikation B enthielt wurde im Verhältnis 1 : 0.8 in Isopropanol dispergiert und bei 35 °C gerührt. Bereits nach 70 Minuten Rühren war der charakteristische starke B-Reflex bei 5.5 °2ϑ verschwunden, und es wurden nur noch die charakteristischen Reflexe der Modifikation A bei 6.9 und 9.2 °2ϑ beobachtet.

### 5. Beispiel

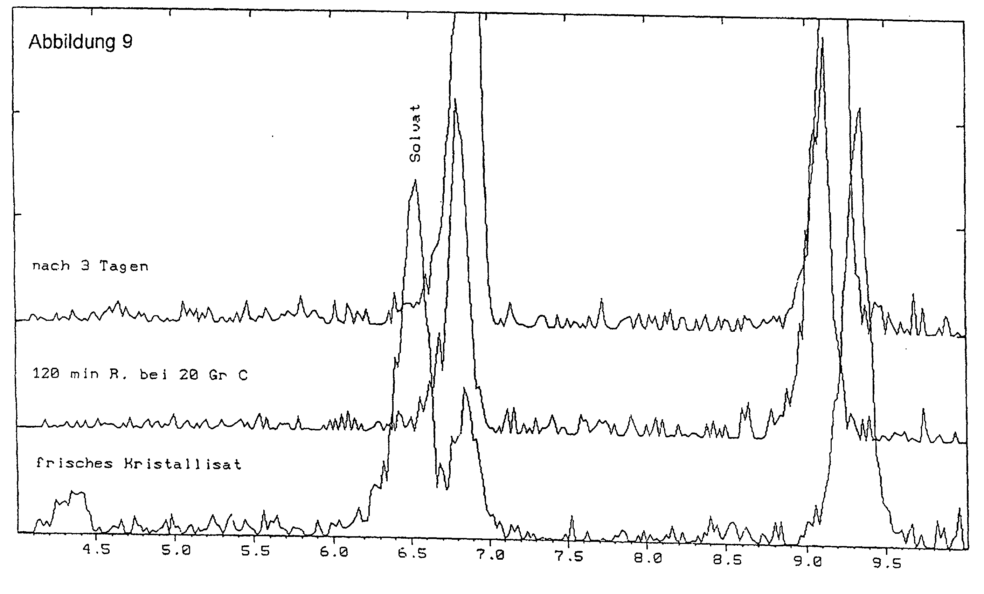

Etwas weniger als die maximal lösliche Menge Flupirtin-Maleat wurde bei 60 °C vollständig in Isopropanol gelöst, langsam bis auf 20 °C abgekühlt und anschließend bei 20 °C gerührt. Vor Beginn des Rührens wurde Solvat neben Modifikation A beobachtet (Abbildung 9, unterste Kurve). Nach 120 Minuten war nur noch die Modifikation A nachweisbar (Abbildung 9, mittlere Kurve). Eine Rückbildung von Solvat wurde auch nach 3 Tagen Stehenlassen der Suspension nicht beobachtet (Abbildung 9. oberste Kurve).

### 6. Beispiel

Etwas weniger als die maximal lösliche Menge Flupirtin-Maleat wurde bei 60 °C vollständig in Isopropanol, das 5 % Wasser enthielt, gelöst, bis auf 20 °C abgekühlt und anschließend bei 20 °C gerührt. Vor Beginn des Rührens lag Solvat neben Modifikation A vor. Nach 130 Minuten Rühren war nur noch die Modifikation A nachweisbar.

### 7. Beispiel

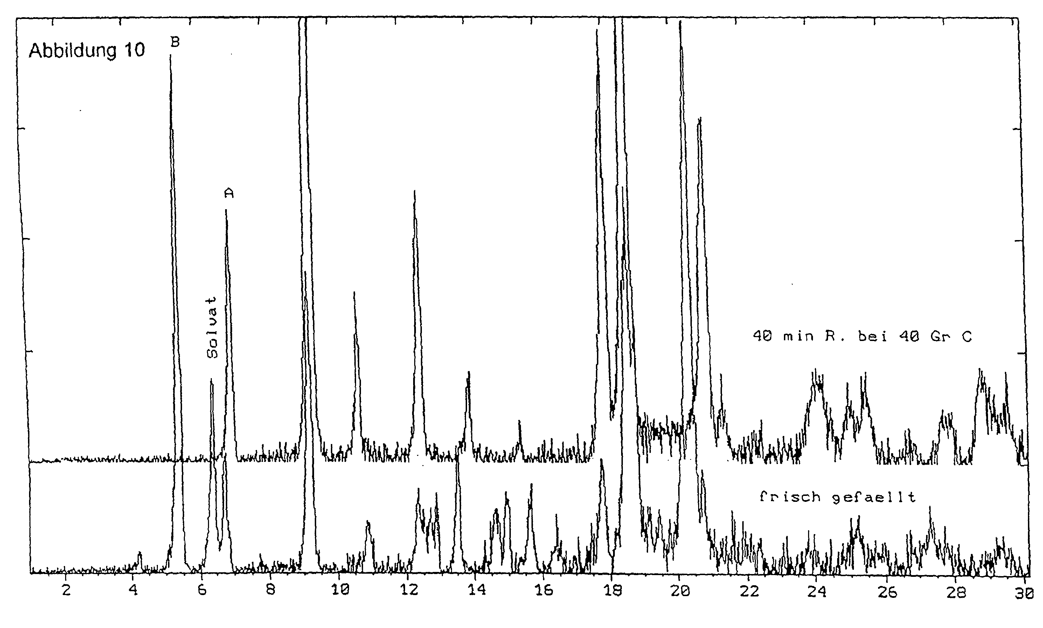

Jeweils stöchiometrische Mengen von Flupirtin-Base und Maleinsäure wurden bei 50 °C in Isopropanol gelöst. Die Fällung des Flupirtin-Maleates erfolgte durch Eintropfen der Maleinsäurelösung in die Lösung der Base bei 40 °C. Das frisch gefällte Kristallisat enthielt Solvat neben der Modifikation B (Abbildung 10, untere Kurve). Bereits nach 40 Minuten Rühren bei 40 °C waren sowohl das Solvat als auch die Modifikation B vollständig in die Modifikation A umgewandelt (Abbildung 10, obere Kurve).

### 8. Beispiel

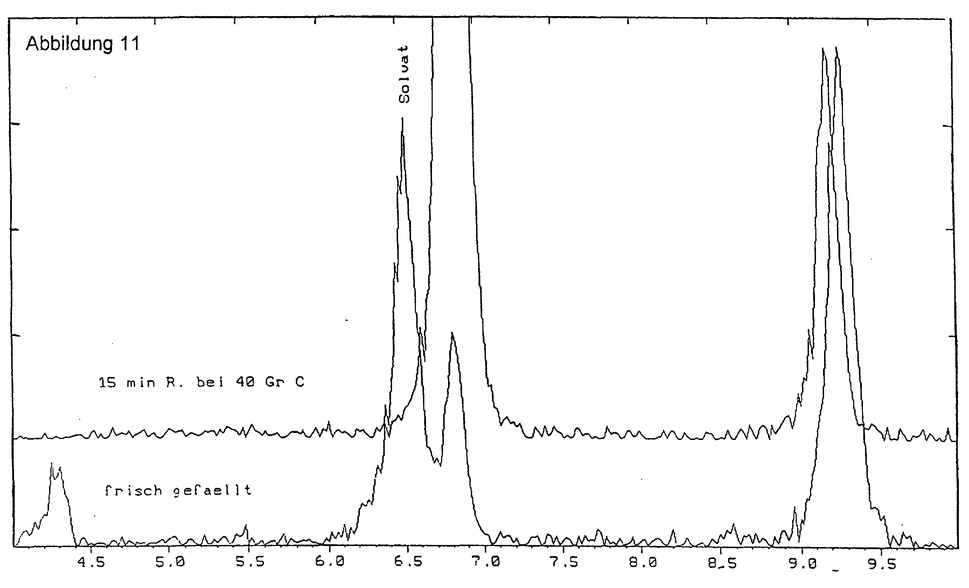

Jeweils stöchiometrische Mengen von Flupirtin-Base und Maleinsäure wurden bei 40 °C in Ethanol gelöst. Die Fällung des Flupirtin-Maleates erfolgte durch Eintropfen der Maleinsäurelösung in die Lösung der Base bei 40 °C. Im frischgefällten Kristallisat war nur Solvat nachweisbar (Abbildung 11, untere Kurve). Bereits nach 15 Minuten Rühren bei 40 °C war das Solvat vollständig in die Modifikation A umgewandelt (Abbildung 11, obere Kurve).

## Patentansprüche

1. Verfahren zur Herstellung von reinem Flupirtin-Maleat durch Hydrierung von 2-Amino-3-nitro-6-(4-fluorbenzyl-amino)-pyridin (ANFP) in Gegenwart von Raney-Nickel, Acylierung mit Chlorameisenessigsäureethylester und die Umsetzung der erhaltenen Flupirtin-Base mit Maleinsäure **dadurch gekennzeichnet, daß** die Hydrierung, Acylierung und Fällung in wasserlöslichen Alkoholen erfolgt.

2. Verfahren nach Anspruch 1 **dadurch gekennzeichnet, daß** als Alkohole vorzugsweise Ethanol oder Isopropanol eingesetzt werden.

3. Verfahren nach Anspruch 1 **dadurch gekennzeichnet, daß** entweder
a) das Flupirtin-Rohmaleat isoliert und durch Kristallisation in in das reine Maleat überführt oder
b) aus der in situ gebildeten Flupirtin-Base durch Umsetzung mit Maleinsäure das Maleat gefällt und ohne Isolierung in das reine Flupirtin-Maleat überführt oder
c) die Flupirtin-Rohbase gefällt und umkristallisiert und anschließend durch Umsetzung mit Maleinsäure in Flupirtin-Reinmaleat überführt wird.

4. Verfahren nach den Ansprüchen 1 bis 3 **dadurch gekennzeichnet, daß** die Umsetzung bei einer Temperatur zwischen -10 - 60 °C durchgeführt wird.

5. Verfahren zur Herstellung der reinen Modifikation A des Flupirtin-Maleates **dadurch gekennzeichnet, daß**,das Flupirtin-Maleat, hergestellt nach den Ansprüchen 1 bis 3, in gegebenenfalls wasserhaltigem Isopropanol oder Ethanol gerührt wird.

6. Verfahren nach Anspruch 5 **dadurch gekennzeichnet, daß** der Rührprozeß vorzugsweise in der Reaküonsmischung gemäß Anspruch 3 c), enthaltend auskristallisiertes Flupirtin-Maleat, durchgeführt wird.

7. Verfahren nach Anspruch 5 **dadurch gekennzeichnet, daß** der Rührprozeß bei Temperaturen zwischen -10 bis 60 °C durchgeführt wird.

8. Reine Modifikation A des Flupirtin-Maleates, erhältlich durch das Verfahren nach Anspruch 5.

## Claims

1. Process for the preparation of pure flupirtine maleate by hydrogenation of 2-amino-3-nitro-6-(4-fluorobenzylamino)pyridine (ANFP) in the presence of Raney nickel, acylation with ethyl chloroformate and reaction of the resulting flupirtine base with maleic acid, **characterized in that** the hydrogenation, acylation and precipitation are carried out in water-soluble alcohols.

2. Process according to Claim 1, **characterized in that** the alcohols used are preferably ethanol or isopropanol.

3. Process according to Claim 1, **characterized in that** either
a) the crude flupirtine maleate is isolated and is converted into the pure maleate by crystallization or
b) the maleate is precipitated by reacting the flupirtine base formed in situ with maleic acid and is converted into the pure flupirtine maleate without isolation or
c) the crude flupirtine base is precipitated and recrystallized and then converted into pure flupirtine maleate by reaction with maleic acid.

4. Process according to any of Claims 1 to 3, **characterized in that** the reaction is carried out at a temperature between -10 and 60°C.

5. Process for the preparation of the pure A modification of flupirtine maleate, **characterized in that** the flupirtine maleate prepared according to any of Claims 1 to 3 is stirred in optionally water-containing isopropanol or ethanol.

6. Process according to Claim 5, **characterized in that** the stirring process is preferably carried out in the reaction mixture according to Claim 3 c), containing flupirtine maleate which has crystallized out.

7. Process according to Claim 5, **characterized in that** the stirring process is carried out at temperatures between -10 and 60°C.

8. Pure A modification of flupirtine maleate, obtainable by the method according to Claim 5.

## Revendications

1. Procédé de fabrication de maléate de flupirtine pur, dont la purification est effectuée par hydrogénation de la 2-amino-3-nitro-6-(4-fluorbenzyl-amino)-pyridine (ANFP) en présence de nickel de Raney, par une acylation utilisant du chloroformate d'éthyle et par réaction entre la base de flupirtine obtenue et l'acide maléique,
**caractérisé en ce que**
l'hydrogénation, l'acylation et la précipitation se font dans des alcools solubles dans l'eau.

2. Procédé selon la revendication 1,
**caractérisé en ce que**
les alcools préférés sont l'éthanol ou l'isopropanol.

3. Procédé selon la revendication 1,
**caractérisé en ce que**
a) le maléate de flupirtine brut est séparé de la solution et i est converti en maléate de flupirtine pur par cristallisation ou
b) in situ, le maléate précipité, après réaction de la base de la flupirtine avec l'acide maléfique, est converti en maléate de flupirtine pur sans séparation de la solution ou
c) la base de flupirtine est précipitée, recristallisée, et est ensuite convertie en maléate de flupirtine pur par réaction avec l'acide maléique.

4. Procédé selon les revendications 1 à 3,
**caractérisé en ce que**
le procédé est mis en oeuvre à des températures comprises entre -10 à 60°C.

5. Procédé de fabrication de modification A du maléate de flupirtine pur,
**caractérisé en ce que**
le maléate de flupirtine pur est fabriqué selon les revendications 1 à 3 dans l'isopropanol ou l'éthanol éventuellement aqueux.

6. Procédé selon la revendication 5,
**caractérisé en ce que**
le processus d'agitation est mis en oeuvre de préférence, conformément à la revendication 3 c), dans le mélange réactionnel contenant le maléate de flupirtine cristallisé.

7. Procédé selon la revendication 5,
**caractérisé en ce que**
le processus d'agitation est mis en oeuvre à des températures comprises entre -10 et 60°C.

8. Modification A pure du maléate de flupirtine, obtenu par le procédé selon la revendication 5.
